# EUROPEAN PATENT APPLICATION

(11) **EP 4 715 829 A1**
(43) Date of publication of application: **25.03.2026**
(21) Application number: 24807200.1
(22) Date of filing: 14.05.2024
(51) Int. Cl.: G16C 20/70, G16C 20/30

(54) **DESIGN DEVICE, DESIGN METHOD, PROGRAM, AND DESIGN SYSTEM**

(30) Priority: 16.05.2023 JP 2023080836
(71) Applicant: Resonac Corporation, Tokyo 105-7325 (JP)
(72) Inventor: ASOMA, Yuichiro, Tokyo 105-7325 (JP); HIRAKAWA, Teruo, Tokyo 105-7325 (JP); OKUNO, Yoshishige, Tokyo 105-7325 (JP)
(74) Representative: Strehl & Partner mbB
(86) International application number: PCT/JP2024/017744
(87) International publication number: WO 2024/237248

(57) **Abstract**

A design device for designing a classical force field includes an inference unit configured to infer, using a deep learning force field that has learned results of a first-principles calculation of a selected chemical structure, physical property values of the chemical structure necessary for creating the classical force field; and a determination unit configured to determine parameters of a function system of the classical force field, using the inferred physical property values of the chemical structure.

## Description

### TECHNICAL FIELD

The present disclosure relates to a design device, a design method, a program, and a design system.

### BACKGROUND

With the development of computing technology in recent years, predictions of a wide variety of physical properties, such as chemical reactions or chemical adsorption, have become possible, using various force fields (sets of simulation functions and parameters). Here, although the force field has various features, including target chemical species and their effects, because all of them are specialized in a specific system, rapid development of a general-purpose force field is required.

For example, Patent Document 1 describes neural network potential (NNP) as a method for realizing inference based on results obtained by first-principles calculation.

### Related Art Document

### Patent Document

[Patent Document 1] Japanese Laid-open Patent Application Publication No. 2022-189626

### SUMMARY OF THE INVENTION

### Problem to be solved by the invention

For the behavior analysis of chemical reaction or chemical adsorption, for example, first-principles calculation or quantum chemical calculation is used. First-principles calculation or quantum chemical calculation has high accuracy and versatility, but has a problem that computational cost is very high. For example, there is a case where first-principles calculation or quantum chemical calculation has a practical limit of about 100 atoms.

Additionally, a simulation using a classical force field, which is an example of the force field, has a very low computational cost and can be used to analyze macromolecules or proteins with about one million atoms. However, a classical force field has a problem that the learning cost is high and versatility is low because first-principles calculation is performed many times to determine parameters of a function system specialized in a target system.

Furthermore, a simulation using a deep learning force field, which is an example of the force field, reproduces first-principles calculation results having high accuracy and versatility by deep learning. A trained deep learning force field has high accuracy and versatility. However, a deep learning force field has a problem that computational cost or memory utilization of the deep learning force field does not reach that of the classical force field.

It is an object of the present disclosure to provide a design device, a design method, a program, and a design system configured to support the design of a classical force field.

### Means for Solving the Problem

The present disclosure has the following configurations.
[1] A design device for designing a classical force field, including:
   an inference unit configured to infer, using a deep learning force field that has learned results of a first-principles calculation of a selected chemical structure, physical property values of the chemical structure necessary for creating the classical force field; and
   a determination unit configured to determine parameters of a function system of the classical force field, using the inferred physical property values of the chemical structure.
[2] The design device as described in [1], wherein the determination unit determines the parameters by fitting the inferred physical property values of the chemical structure to the function system of the classical force field.
[3] The design device as described in [1] or [2], wherein the deep learning force field is a neural network used for neural network potential (NNP), and has been trained to output the results of first-principles calculation of the chemical structure when the chemical structure is input.
[4] The design device as described in any one of [1] to [3], wherein the physical property values of the chemical structure are energies when each of a bond length, bond angle, and dihedral angle is changed.
[5] A design method performed by a design device for designing a classical force field, the design method including:
   an inference procedure of inferring, using a deep learning force field that has learned results of a first-principles calculation of a selected chemical structure, physical property values of the chemical structure necessary for creating the classical force field; and
   a determination procedure of determining parameters of a function system of the classical force field, using the inferred physical property values of the chemical structure.
[6] A program causing a design device for designing a classical force field to perform:
   an inference step of inferring, using a deep learning force field that has learned results of a first-principles calculation of a selected chemical structure, physical property values of the chemical structure necessary for creating the classical force field; and
   a determination step of determining parameters of a function system of the classical force field, using the inferred physical property values of the chemical structure.
[7] A design system for designing a classical force field, including:
   a first-principles calculation unit configured to perform a first-principles calculation;
   a learning unit configured to cause a deep learning force field to perform learning, using results of the first-principles calculation unit performing the first-principles calculation on a selected chemical structure;
   an inference unit configured to infer, using the deep learning force field that has learned the results of the first-principles calculation, physical property values of the chemical structure necessary for creating the classical force field; and
   a determination unit configured to determine parameters of a function system of the classical force field, using the inferred physical property values of the chemical structure.

### Effect of the invention

According to the present disclosure, a design device, a design method, a program, and a design system configured to support the design of a classical force field can be provided.

### BRIEF DESCRIPTION OF THE DRAWINGS

[FIG. 1] FIG. 1 is a configuration diagram of an example of a design system according to the present embodiment.
[FIG. 2] FIG. 2 is a hardware configuration diagram of an example of a computer according to the present embodiment.
[FIG. 3] FIG. 3 is a functional configuration diagram of an example of a design system according to the present embodiment.
[FIG. 4A] FIG. 4A is a diagram for explaining an example of a method of creating a classical force field.
[FIG. 4B] FIG. 4B is a diagram for explaining an example of the method of creating the classical force field.
[FIG. 4C] FIG. 4C is a diagram for explaining an example of the method of creating the classical force field.
[FIG. 5] FIG. 5 is a flowchart of an example of a process of creating a deep learning force field according to the present embodiment.
[FIG. 6] FIG. 6 is a flowchart of an example of a process of creating the classical force field according to the present embodiment.

### DESCRIPTION OF THE EMBODIMENTS

Next, embodiments of the present invention will be described in detail. Here, the present invention is not limited to the following embodiments.

### [First Embodiment]

### <System Configuration>

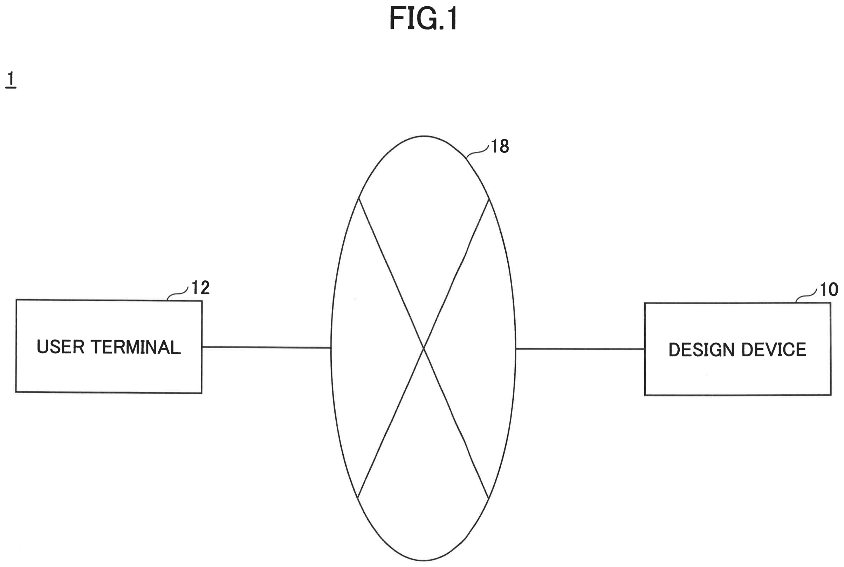

FIG. 1 is a configuration diagram of an example of a design system 1 according to the present embodiment. The design system 1 of FIG. 1 includes a design device 10 and a user terminal 12. The design device 10 and the user terminal 12 of the design system 1 are connected to each other via a communication network 18, such as a local area network (LAN) or the Internet, to enable data communication.

The user terminal 12 is an information processing terminal, such as a PC, a tablet terminal, or a smartphone, operated by an operator. The user terminal 12 displays a screen for accepting input of information from the operator on a display device and accepts the input of the information from the operator. Additionally, the user terminal 12 transmits, to the design device 10, the information accepted from the operator to cause the design device 10 to perform processing that supports the design of a classical force field. The user terminal 12 receives information on a result of the design device 10 performing the processing and displays it on the display device to make the operator check it.

The design device 10 is an information processing device, such as a PC or a workstation, that supports the design of the classical force field by the operator. The design device 10 performs the calculation of data sampling required for parameter fitting of a function system of the classical force field, not directly by the first-principles calculation but by using a deep learning force field that has learned results of the first-principles calculation, thereby increasing the speed of the calculation of the data sampling. The design device 10 receives the information input by the operator to the user terminal 12 and performs the processing that supports the design of the classical force field. The design device 10 transmits the result information on the processing to the user terminal 12 and causes the user terminal 12 to display the result information on the processing.

The design system 1 of FIG. 1 may be realized by the design device 10 having a Web server function and the user terminal 12 executing a Web application by a Web browser function. Additionally, the design system 1 of FIG. 1 may be realized by the application installed in the user terminal 12 performing the processing in cooperation with the program installed in the design device 10.

Here, the design system 1 in FIG. 1 is merely an example, and it is needless to say that there are various system configuration examples according to applications and purposes. For example, the design device 10 may be realized by a plurality of computers or may be realized as a cloud computing service. The design system 1 may be realized by a stand-alone computer.

### <Hardware Configuration>

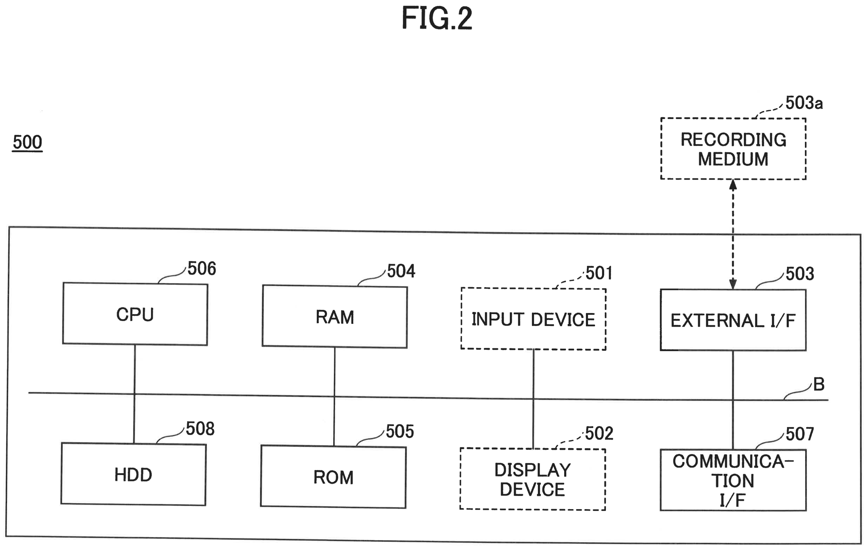

The design device 10 and the user terminal 12 of FIG. 1 are realized by a computer 500 having a hardware configuration illustrated in FIG. 2, for example.

FIG. 2 is a hardware configuration diagram of an example of the computer 500 according to the present embodiment. The computer 500 of FIG. 2 includes an input device 501, a display device 502, an external I/F 503, a RAM 504, a ROM 505, a CPU 506, a communication I/F 507, an HDD 508, and the like, which are mutually connected by a bus B. Here, the input device 501 and the display device 502 may be configured to be connected for use.

The input device 501 includes a touch panel, operation keys and buttons, a keyboard and a mouse, and the like, which are used by the operator to input various signals. The display device 502 includes a display, such as a liquid crystal display or an organic EL display, for displaying a screen, a speaker for outputting sound data, such as voice and sound, and the like. The communication I/F 507 is an interface for the computer 500 to perform data communication.

Additionally, the HDD 508 is an example of a nonvolatile storage device for storing programs and data. The stored programs and data include an OS, which is basic software for controlling the entire computer 500, applications for providing various functions on the OS, and the like. Here, instead of the HDD 508, the computer 500 may use a drive device using a flash memory as a storage medium (for example, a solid-state drive: SSD or the like).

The external I/F 503 is an interface with an external device. The external device includes a recording medium 503a and the like. With this, the computer 500 can read from and/or write to the recording medium 503a via the external I/F 503. The recording medium 503a includes a flexible disk, a CD, a DVD, an SD memory card, a USB memory, and the like.

The ROM 505 is an example of a nonvolatile semiconductor memory (a storage device) that can hold programs and data even when the power is turned off. The ROM 505 stores programs and data, such as BIOS, OS settings, and network settings that are executed when the computer 500 is started. The RAM 504 is an example of a volatile semiconductor memory (a storage device) that temporarily holds programs and data.

The CPU 506 is an arithmetic unit that reads programs and data from the storage device, such as the ROM 505 and the HDD 508, into the RAM 504 and performs the processing, thereby realizing control and functions of the entire computer 500. The computer 500 according to the present embodiment can realize various functions of the design device 10 and the user terminal 12, which will be described later, by executing programs.

Here, a program to be installed in the RAM 504 is installed by a program recorded in the recording medium 503a being read via the external I/F 503, for example. Alternatively, the program may be installed by being downloaded from the communication network 18 via the communication I/F 507.

### <Functional Configuration>

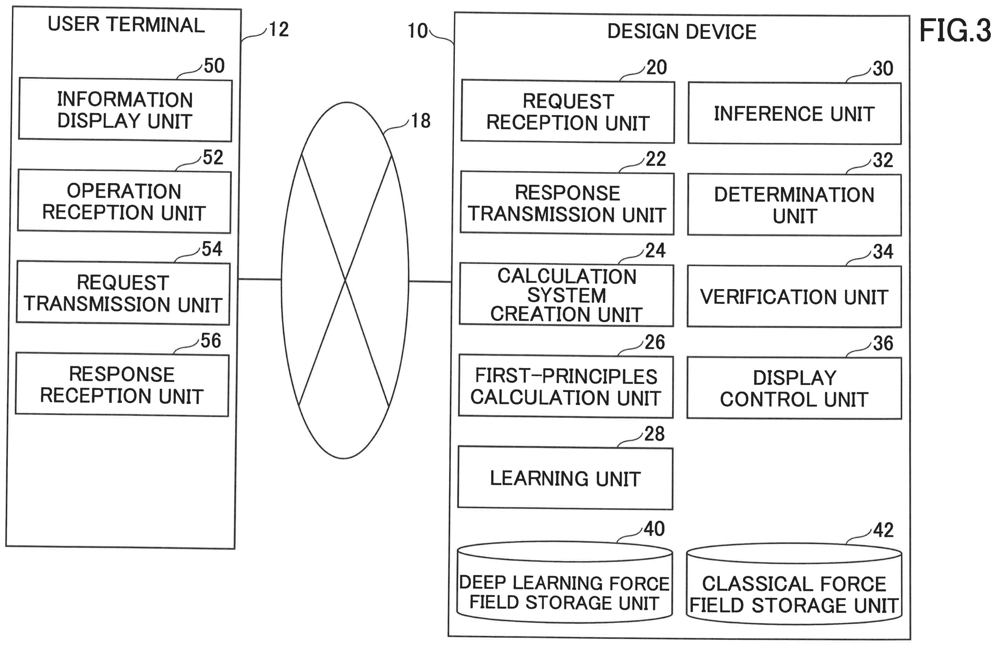

A functional configuration of the design system 1 according to the present embodiment will be described. FIG. 3 is a functional configuration diagram of an example of the design system 1 according to the present embodiment. Here, in the configuration diagram of FIG. 3, portions unnecessary for the description of the present embodiment are appropriately omitted.

The design device 10 of the design system 1 illustrated in FIG. 3 includes a request reception unit 20, a response transmission unit 22, a calculation system creation unit 24, a first-principles calculation unit 26, a learning unit 28, an inference unit 30, a determination unit 32, a verification unit 34, a display control unit 36, a deep learning force field storage unit 40, and a classical force field storage unit 42. The user terminal 12 of the design system 1 includes an information display unit 50, an operation reception unit 52, a request transmission unit 54, and a response reception unit 56.

The information display unit 50 displays, on the display device 502, a screen for accepting input of information from the operator and the information on the result of performing the processing of the design device 10. The operation reception unit 52 accepts an operation of the operator such as input of information. The request transmission unit 54 transmits, to the design device 10, a request for processing corresponding to the input of the information from the operator. Additionally, the response reception unit 56 receives, from the design device 10, a response to the request for the processing transmitted by the request transmission unit 54.

The request reception unit 20 receives the request for the processing from the user terminal 12. The response transmission unit 22 responds with the result of performing the processing corresponding to the request for the processing. The calculation system creation unit 24 creates a calculation system using a selected chemical structure based on information of the selected chemical structure input from the operator. The first-principles calculation unit 26 performs a first-principles calculation on structural models in which each of a bond length, a bond angle, and a dihedral angle of the selected chemical structure is changed, and calculates the energies of the structural models as the results of the first-principles calculation. The bond length, bond angle, and dihedral angle are examples of physical property values of the chemical structure. The first-principles calculation is a calculation based on, for example, density function theory (DFT) or quantum mechanics (QM).

The learning unit 28 causes the deep learning force field to learn a relationship between the structural models in which each of the bond length, bond angle, and dihedral angle of the selected chemical structure is changed and the energies of the structural models calculated as the results of the first-principles calculation, based on an appropriate machine learning method. With this, the machine learning of the deep learning force field may be performed using the selected chemical structure and the physical property values of the chemical structure calculated by the first-principles calculation for the selected chemical structure as training data.

Additionally, the deep learning force field is a neural network used for neural network potential (NNP), and has been trained to output, when a chemical structure is input, a result of a first-principles calculation of the chemical structure. The trained deep learning force field can infer a result of the first-principles calculation for the input chemical structure. The learning unit 28 stores the trained deep learning force field in the deep learning force field storage unit 40. The deep learning force field storage unit 40 stores the trained deep learning force field. The deep learning force field storage unit 40 may store a trained deep learning force field open to the Internet or the like.

The inference unit 30 uses the trained deep learning force field stored in the deep learning force field storage unit 40 to infer energies of the structure model as the results of the first-principles calculation for the structure models in which each of bond length, bond angle, and dihedral angle of a selected chemical structure is changed.

The determination unit 32 fits the energies of the structure model inferred by the inference unit 30 to the function system of the classical force field, and determines parameters of the classical force field. The determination unit 32 generates the classical force field by determining the parameters of the function system of the classical force field. Details of the processing of the determination unit 32 will be described later. The classical force field storage unit 42 stores the generated classical force field.

The verification unit 34 verifies whether the parameters of the function system of the classical force field determined by the determination unit 32 are appropriate. The display control unit 36 controls the display of the user terminal 12. Here, the functional configuration diagram of FIG. 3 is an example. The design system 1 according to the present embodiment can be realized by various configurations. For example, the deep learning force field storage unit 40 and the classical force field storage unit 42 may be included in a storage device, a computer, or a cloud storage with which the design device 10 can perform data communication.

### <Processing>

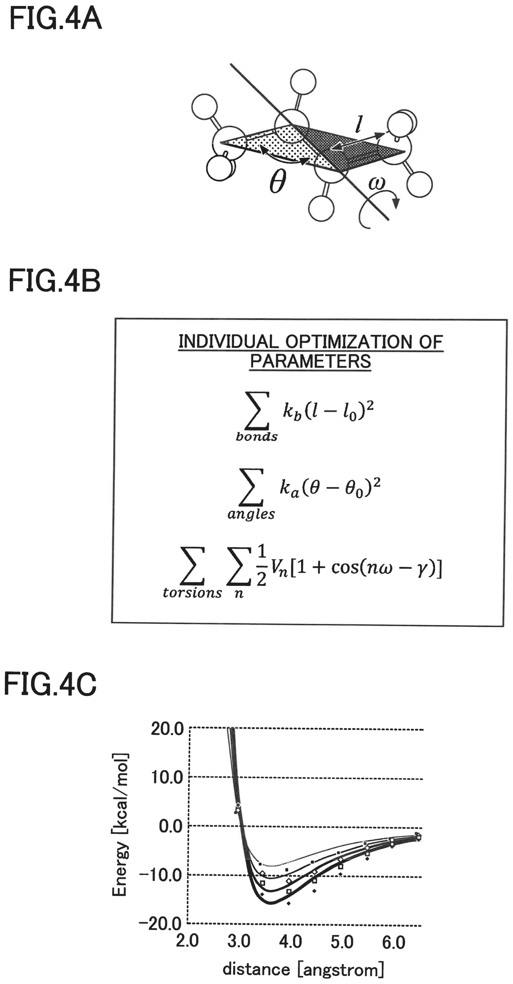

FIGS. 4A to 4C are diagrams for explaining an example of a method of creating the classical force field. FIG. 4A is a schematic diagram of an example of a calculation system created using a selected chemical structure. "l" in FIG. 4A represents the bond length between two particles. "θ" in FIG. 4A represents the bond angle between three particles. "ω" in FIG. 4A represents the dihedral angle between four particles. FIG. 4B illustrates an example of a function system of a classical force field for the chemical structure illustrated in FIG. 4A. The function system of the classical force field includes parameters k_{b}, kₐ, and Vₙ.

In FIG. 4C, for example, the energy is plotted when the bond length l is changed in steps of 0.5 [angstroms]. The plotting is performed by the calculation of the data sampling. In the creation of the classical force field, the parameters of the function system of the classical force field are determined by individual optimization of the parameters in FIG. 4B so that the function system approximates the plots in FIG. 4C.

Here, the individual optimization of the parameters in FIG. 4B may be performed by processing of a program for determining the parameters using, for example, the least squares method, so that the difference from the plots in FIG. 4C is minimized, or may be performed by the operator adjusting the parameters.

Here, when the first-principles calculation is performed for the plots in FIG. 4C, it is necessary to perform the first-principles calculations of the number of plots. The first-principles calculation has high accuracy and versatility, but the computational cost is very high. Thus, when the first-principles calculation is performed for the plotting in FIG. 4C, the computational cost is high. Therefore, in the present embodiment, for the plotting in FIG. 4C, the result of the first-principles calculation is simulated using the trained deep learning force field. The simulation using the deep learning force field reproduces the results of the first-principles calculation with high accuracy. The trained deep learning force field has a lower computational cost than the first-principles calculation. Therefore, when the simulation using the trained deep learning force field is performed for the plotting in FIG. 4C, the computational cost is lower than the computational cost of performing the first-principles calculation. Additionally, when the simulation using the trained deep learning force field is performed for the plotting in FIG. 4C, the memory utilization is superior to that of the first-principles calculation. If the memory utilization is superior, the number of atoms that can be calculated can be expected to increase.

Additionally, as for the deep learning force field, a published deep learning force field can be used, and, for example, a published deep learning force field in the Open Catalyst Project can be used. By using the published deep learning force field, the learning cost of the deep learning force field can be reduced in the present embodiment.

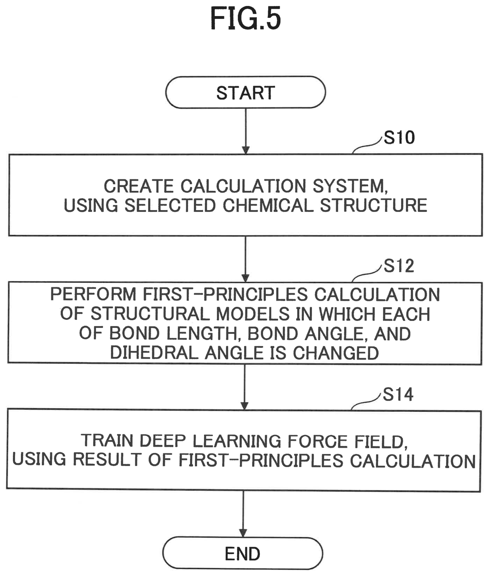

If there is no published deep learning force field, in the present embodiment, for example, a deep learning force field is created by the procedure illustrated in FIG. 5. FIG. 5 is a flowchart of an example of a process of creating the deep learning force field according to the present embodiment.

In step S10, the calculation system creation unit 24 creates a calculation system using a selected chemical structure, for example, as illustrated in FIG. 4A, based on information related to the selected chemical structure input from an operator.

In step S12, the first-principles calculation unit 26 performs the first-principles calculation on the structural models in which each of the bond length, bond angle, and dihedral angle of the selected chemical structure is changed as illustrated in FIG. 4A, for example, and calculates the energies of the structural models as a result of the first-principles calculation as illustrated in the plots in FIG. 4C, for example.

In step S14, the learning unit 28 causes the deep learning force field to learn the relationship between the structural models in which each of the bond length, bond angle, and dihedral angle of the selected chemical structure is changed and the energies of the structural models calculated as the results of the first-principles calculation, based on an appropriate machine learning method.

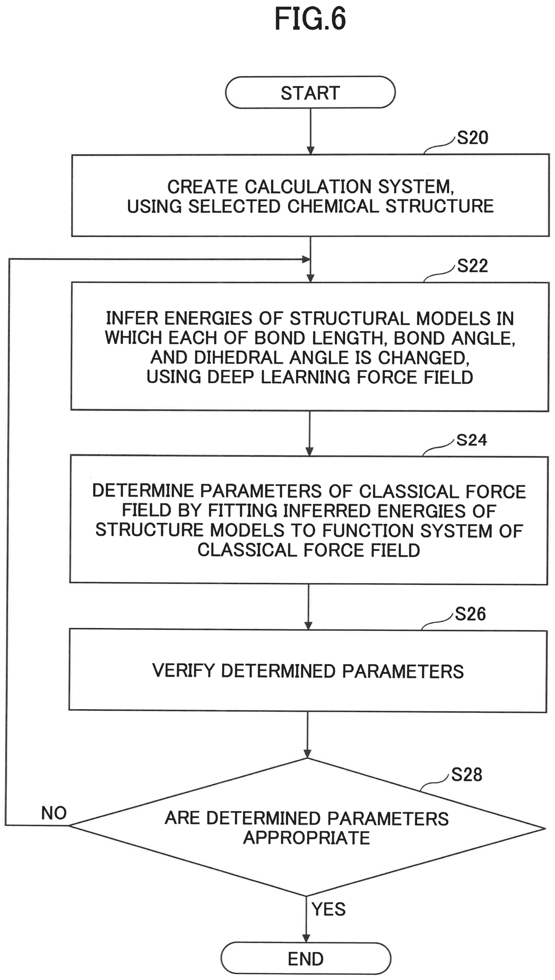

FIG. 6 is a flowchart of an example of a process of creating a classical force field according to the present embodiment.

In step S20, the calculation system creation unit 24 creates the calculation system using the selected chemical structure as illustrated in FIG. 4A, for example, based on the information related to the selected chemical structure input from the operator.

In step S22, the inference unit 30 infers the energies of the structural models in which each of the bond length, bond angle, and dihedral angle of the selected chemical structure is changed, using the trained deep learning force field stored in the deep learning force field storage unit 40, and calculates the energies of the structural models as illustrated in the plots in FIG. 4C, for example.

In step S24, the determination unit 32 determines the parameters of the classical force field by fitting the energies of the structure models inferred by the inference unit 30 in step S22 to the function system of the classical force field. For example, the determination unit 32 determines the parameters of the function system of the classical force field by individually optimizing the parameters of FIG. 4B so that the function system approximates the plots of FIG. 4C.

In step S26, the verification unit 34 verifies whether the parameters determined by the determination unit 32 in step S24 are appropriate. For example, the verification unit 34 performs molecular dynamics calculation by using the parameters determined in step S24 in the function system of the classical force field of FIG. 4B, and verifies whether the parameters are appropriate based on the result.

If the parameters determined in step S24 are not appropriate, the design device 10 returns to the processing of step S22 and continues the process. If the parameters determined in step S24 are appropriate, the design device 10 ends the process of the flowchart of FIG. 6. Here, the processes of the flowcharts of FIGS. 5 and 6 may be performed after separating the selected chemical structure when the selected chemical structure is, for example, a polymer or a protein.

### [Other Embodiments]

The design device 10 according to the present embodiment may have a configuration in which a plurality of computers cooperate to perform the above process. For example, the design device 10 of the design system 1 according to the present embodiment may be implemented by being divided into a device configured to perform a first-principles calculation, a device configured to create a deep learning force field, and a device configured to design a classical force field.

As described above, according to the design system 1 of the present embodiment, the speed of the calculation of data sampling required for fitting the parameters of the function system of the classical force field can be increased, and the parameters of the function system of the classical force field can be efficiently determined. Therefore, according to the present embodiment, a design device, a design method, a program, and a design system configured to support the efficient design of the classical force field can be provided.

Although the present embodiments have been described above, it will be understood that various changes in form and details are possible without departing from the spirit and scope of the claims. Although the present invention has been described based on the embodiments, the present invention is not limited to the above embodiments, and various modifications are possible within the scope of the claims. This application claims priority to basic application No. 2023 - 080836, filed on May 16, 2023 in the Japan Patent Office, the entire contents of which are incorporated herein by reference.

### Description of reference symbols

- 1: design system
- 10: design device
- 12: user terminal
- 18: communication network
- 24: calculation system creation unit
- 26: first-principles calculation unit
- 28: learning unit
- 30: reasoning unit
- 32: determination unit
- 34: verification unit

## Claims

1. A design device for designing a classical force field, comprising:
an inference unit configured to infer, using a deep learning force field that has learned results of a first-principles calculation of a selected chemical structure, physical property values of the chemical structure necessary for creating the classical force field; and
a determination unit configured to determine parameters of a function system of the classical force field, using the inferred physical property values of the chemical structure.

2. The design device as claimed in claim 1, wherein the determination unit determines the parameters by fitting the inferred physical property values of the chemical structure to the function system of the classical force field.

3. The design device as claimed in claim 1 or 2, wherein the deep learning force field is a neural network used for neural network potential (NNP), and has been trained to output the results of first-principles calculation of the chemical structure when the chemical structure is input.

4. The design device as claimed in any one of claims 1 to 3, wherein the physical property values of the chemical structure are energies when each of a bond length, bond angle, and dihedral angle is changed.

5. A design method performed by a design device for designing a classical force field, the design method comprising:
an inference procedure of inferring, using a deep learning force field that has learned results of a first-principles calculation of a selected chemical structure, physical property values of the chemical structure necessary for creating the classical force field; and
a determination procedure of determining parameters of a function system of the classical force field, using the inferred physical property values of the chemical structure.

6. A program causing a design device for designing a classical force field to perform:
an inference step of inferring, using a deep learning force field that has learned results of a first-principles calculation of a selected chemical structure, physical property values of the chemical structure necessary for creating the classical force field; and
a determination step of determining parameters of a function system of the classical force field, using the inferred physical property values of the chemical structure.

7. A design system for designing a classical force field, comprising:
a first-principles calculation unit configured to perform a first-principles calculation;
a learning unit configured to cause a deep learning force field to perform learning, using results of the first-principles calculation unit having performed the first-principles calculation on a selected chemical structure;
an inference unit configured to infer, using the deep learning force field that has learned the results of the first-principles calculation, physical property values of the chemical structure necessary for creating the classical force field; and
a determination unit configured to determine parameters of a function system of the classical force field, using the inferred physical property values of the chemical structure.
